# EUROPEAN PATENT SPECIFICATION

(11) **EP 4 404 920 B1**
(45) Date of publication and mention of the grant of the patent: **15.07.2026**
(21) Application number: 22793885.9
(22) Date of filing: 21.09.2022
(51) Int. Cl.: A61K 9/20, A61K 9/16, A61K 47/32, A61K 47/36, A61K 31/36, A61K 31/4025, A61K 31/7048, A61P 35/00

(54) **PHARMACEUTICAL COMPOSITION AND A PROCESS TO PREPARE THE SAME**
PHARMAZEUTISCHE ZUSAMMENSETZUNG UND VERFAHREN ZU IHRER HERSTELLUNG
COMPOSITION PHARMACEUTIQUE ET SON PROCÉDÉ DE PRÉPARATION

(30) Priority: 22.09.2021 IN 202121042945
(43) Date of publication of application: 31.07.2024
(73) Proprietor: Godavari Biorefineries Limited, Fort, Mumbai 400 001 MAH (IN)
(72) Inventor: REDASANI, Vijayendra, Thane West, Maharashtra (IN); GAVADE, Sandip, Mumbai - 400 001, Maharashtra (IN); ATHAVALE, Maithili, Mumbai - 400 001, Maharashtra (IN); KHARKAR, Prashant, Mumbai - 400019, Maharashtra (IN)
(74) Representative: Keltie LLP
(86) International application number: PCT/IN2022/050842
(87) International publication number: WO 2023/047413

(56) References cited:
- WO-A1-2015/153653
- WO-A1-2019/220282
- WO-A2-2018/193476
- US-A1- 2007 196 396
- CRAIG D Q M ET AL: "The relevance of the amorphous state to pharmaceutical dosage forms: Glassy drugs and freeze dried systems", INTERNATIONAL JOURNAL OF PHARMACEUTICS, ELSEVIER, NL, vol. 179, no. 2, 15 March 1999 (1999-03-15), pages 179 - 207, XP002274233, ISSN: 0378-5173, DOI: 10.1016/S0378-5173(98)00338-X

## Description

### FIELD OF THE INVENTION

The present invention relates to a pharmaceutical composition comprising compound of Formula IV, along with a solubilizing agent and a process to prepare the same. The said pharmaceutical composition has enormous applications in cancer therapy

### BACKGROUND OF THE INVENTION

Solubility, the phenomenon of dissolution of solute in solvent to give a homogenous system, is one of the important parameters to achieve desired concentration of drug in systemic circulation for desired pharmacological response. Low aqueous solubility is the major problem encountered with formulation development of new chemical entities as well as for the generic development. More than 40% NCEs (new chemical entities) developed in pharmaceutical industry are practically insoluble in water. The poor solubility and low dissolution rate of poorly water-soluble drugs in the aqueous gastrointestinal fluids cause insufficient bioavailability. The negative effect of compounds with low solubility includes poor absorption and bioavailability, insufficient solubility for IV dosing, development challenges leading to increasing the development cost and time, burden shifted to patient. (10.5402/2012/195727).

Various techniques are used for the enhancement of the solubility of poorly soluble drugs which include physical and chemical modifications of drug and other methods like particle size reduction, crystal engineering, salt formation, solid dispersion, use of surfactant, complexation, and so forth. Selection of solubility improving method depends on drug property, site of absorption, and required dosage form characteristics.

Solubilizing agents, such as surfactants, are solubilizer excipients added to pharmaceutical formulations in order to increase the solubility of poorly soluble drugs, thereby increasing the bioavailability of the corresponding active pharmaceutical ingredient (API). However, it is found that the solubility of poorly water-soluble drugs is way below the desired threshold, which lowers their absorption in the gastrointestinal tract. Also, their stability is lowered considerably. Furthermore, it is well known that poorly water-soluble drugs have reduced drug efficiencies as compared to pharmaceutical formulations with appropriate solubility profiles and may cause undesirable side effects upon administration.

In the BCS (Biopharmaceutics Classification System) classification all drugs have been divided into four classes: class I-high soluble and high permeable, class II-low soluble and high permeable, class III-low soluble and high permeable and class IV-low soluble and low permeable. BCS class II drugs have high absorption number and low dissolution number. The absorption/bioavailability is limited by dissolution rate.

The inventors in the present invention have focused on improving the dissolution of the compounds of Formula IV. The compounds of Formula IV are compounds that demonstrate anticancer and anti-cancer stem cell activity particularly in breast and prostate cancer cell lines. Further, the compounds have also found to be having antiviral property.

The inventors appreciate that the stability of finished pharmaceutical products is also very important. It depends on one hand, on environmental factors such as ambient temperature, humidity and light, and, on the other, on product-related factors, e.g. the chemical and physical properties of the active substance and of pharmaceutical excipients, the dosage form and its composition, the manufacturing process, the nature of the container-closure system and the properties of the packaging materials.

It is a constant challenge to produce a drug product which shows good stability and at the same time has a good dissolution profile which would result in high bioavailability. The present invention addresses this challenge for pharmaceuticals and has come up with a novel and innovative pharmaceutical composition for the compounds of Formula IV that show significant stability as well as the desired bioavailability on drug release. WO 2018/193476 A2 discloses 4-(benzo[d]1,3]dioxol-5-yl)naphthalen-1-ol derivatives for inhibition of uncontrolled cell proliferation, particularly for the treatment of cancer, such as breast and prostate cancer.

### OBJECTS OF THE INVENTION

The object of the invention is to improve the solubility of the compound of Formula IV in a suitable formulation or in a drug product.

Another object of the invention is to improve the stability of the compounds of Formula IV at accelerated conditions.

Another object of the invention is to improve the stability of the compounds of Formula IV in a packed form without degradation of its amorphous form.

Yet another object of the invention is to improve the bioavailability of the compounds of Formula IV.

It is another object of the invention to provide for compositions of the compounds of Formula IV which are highly soluble in physiological buffers.

Another object of the invention is to provide for a pharmaceutical composition of the compounds of Formula IV for the treatment of cancer, especially breast cancer.

Yet another object of the invention is to provide for a solid oral unit dose formulation of the compounds of Formula IV.

Yet another object of the invention is to provide for tablet dosage forms with high drug release of compounds of Formula IV.

It is yet another object of the invention to provide for an environmentally friendly method of preparing pharmaceutical compositions of the compounds of Formula IV.

### SUMMARY OF THE INVENTION

The present invention is directed towards a pharmaceutical composition comprising the compound of Formula IV or its pharmaceutically acceptable salt thereof, 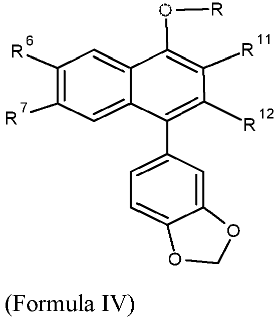 wherein R⁶ and R⁷ each independently is selected from -H, alkoxy, alkyl, -NH₂, -NO₂, -NHCOCH₃, -CN, halogen, -OCF₃ R¹¹ and R¹² each independently is selected from -H, or R¹¹ and R¹² can be 5- or 6-membered ring such as lactone, -C(O)O-alkyl such as -C(O)OC₂H₅; R is selected from 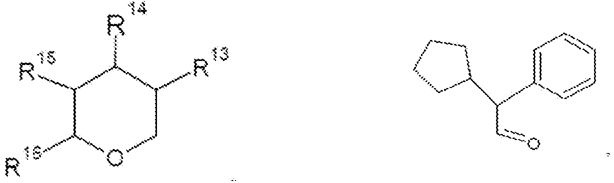 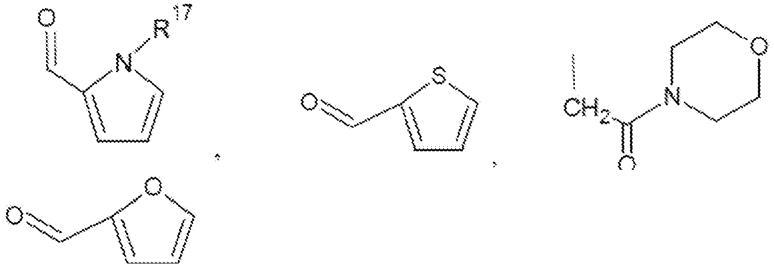 -H, -C(O)CH₂Cl, -SOO-CH₃, -SOOPh, , -CH₂C(O)N(CH₃)₂, -C(O)NHPh, -C(O)NHPhOH, -C(S)NHPh, -CH₂Ph, -COAr, wherein, R¹³ is selected from -OH, -NH₂, -NHCOCH₃, X = F, Cl, Br, alkyl, acetyl, C₃-C₈ acyl group; R¹⁴ is selected from -OMe, -OH, NH₂, -NHCOCH₃, X = F, Cl, Br, alkyl, acetyl, C₃-C₈ acyl group; R¹⁵ is selected from -OMe, -OH, -H, Br, NH₂, X = F, Cl, Br, alkyl, acetyl, C₃-C₈ acyl group; R¹⁶ is selected from -H, -CH₂OH, -OH, alkyl, alkoxy; R¹⁷ is selected from alkyl; along with a solubilizing agent selected from polyvinylpyrrolidone, cyclodextrin, derivative of cyclodextrin, Tween 80, Tween 20, PVPK-30, citric acid, Kolidone VA64, Polyethylene glycol or mixtures thereof, further wherein the compound or a pharmaceutically acceptable salt of Formula IV, in the said pharmaceutical composition is amorphous.

The claimed pharmaceutical composition has improved solubility and drug release for the compounds of Formula IV and at the same time has a very good stability in packed form profile extending up to 18 months not undergoing any degradation in its amorphous form.

The pharmaceutical composition is in a dosage form, preferably in the form of a tablet for oral administration.

### DETAILED DESCRIPTION OF THE INVENTION

The present invention relates to a pharmaceutical composition comprising any of the compound of Formula IV; 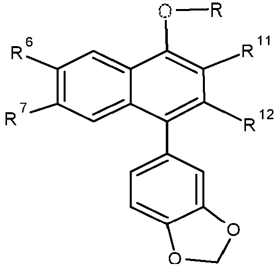 wherein R⁶ and R⁷ each independently is selected from -H, alkoxy, alkyl, NH₂, -NO₂, -NHCOCH₃, -CN, halogen, -OCF₃; R¹¹ and R¹² each independently is selected from -H, or R¹¹ and R¹² can be 5- or 6-membered ring such as lactone, -C(O)O-alkyl such as -C(O)OC₂H₅; R is selected from 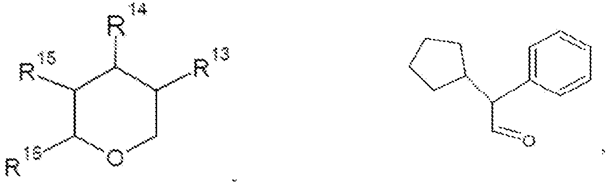 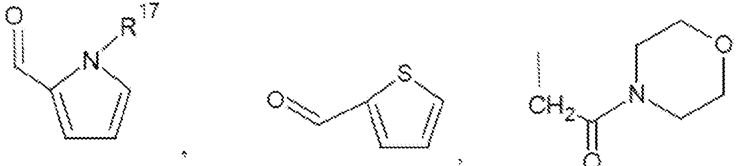 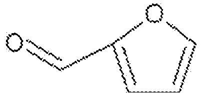 -H, -C(O)CH₂Cl, -SOO-CH₃, -SOOPh, , -CH₂C(O)N(CH₃)₂, -C(O)NHPh, -C(O)NHPhOH, -C(S)NHPh, -CH₂Ph, -COAr, wherein, R¹³ is selected from -OH, -NH₂, -NHCOCH₃, X = F, Cl, Br, alkyl, acetyl, C₃-C₈ acyl group; R¹⁴ is selected from -OMe, -OH, NH₂, -NHCOCH₃, X = F, Cl, Br, alkyl, acetyl, C₃-C₈ acyl group; R¹⁵ is selected from -OMe, -OH, -H, Br, NH₂, X = F, Cl, Br, alkyl, acetyl, C₃-C₈ acyl group; R¹⁶ is selected from -H, -CH₂OH, -OH, alkyl, alkoxy; R¹⁷ is selected from alkyl; along with a solubilizing agent selected from polyvinylpyrrolidone, cyclodextrin, derivative of cyclodextrin, Tween 80, Tween 20, PVPK-30, citric acid, Kolidone VA64, Polyethylene glycol or mixtures thereof, further wherein the compound or a pharmaceutically acceptable salt of any of the compound of Formula IV, in the said pharmaceutical composition is amorphous.

The pharmaceutical compositions are highly stable in a packed form and do not degrade with time. The claimed compositions also shows significant solubility in physiological buffers.

The compounds of the present invention showed poor solubility as currently available in the market. The solubilization capacity of drug compositions are measured in aqueous media which is a primary property of a drug substance. However, it is also critical to understand the solubility in buffer solutions that mimic conditions inside the body such as the gastrointestinal tract, particularly for drugs which are lipophilic and sparingly soluble. The presently claimed composition are surprisingly soluble in multiple buffer solutions. Also, the claimed compositions show excellent stability in accelerated condition. The compositions as claimed further show excellent stability upto 18 months in a packed form and do not degrade with time.

In a preferred embodiment, the compounds of Formula IV are selected from:

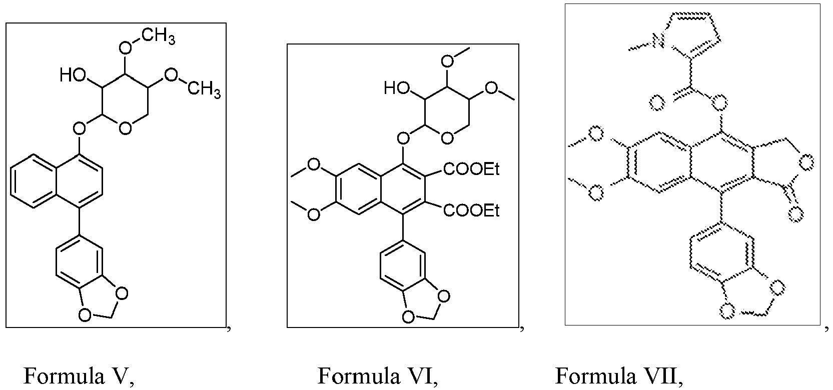

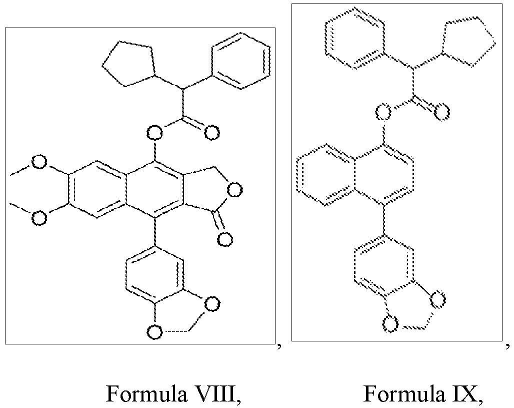

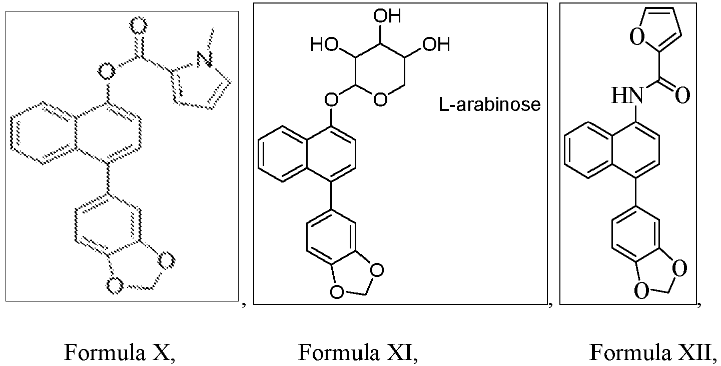

The present invention achieves a drug composition wherein the solubility of the compounds of Formula IV is enhanced in such a manner that it results in increased drug release, and bioavailability. The invention also provides a highly stable composition in accelerated conditions, which simultaneously also achieves enhanced solubility and bioavailability of the compounds of Formula IV.

Therefore, the composition was found to have an improved dissolution profile. The presently claimed composition show good solubility not only in aqueous media but are surprisingly soluble in multiple physiological buffer solutions and at the same time are also stable upto 18 months in a packed form and even in accelerated conditions.

### Definitions

Unless defined otherwise, all technical and scientific terms used herein have the same meaning as commonly understood byone of ordinary skill in the art to which this invention belongs. Although any methods and materials similar or equivalent to those described herein can be used in the practice or testing of the present invention, the preferred methods and materials are described herein. As used herein, each of the following terms has the meaning associated with it in this section. Specific and preferred values listed below for individual process parameters, substituents, and ranges are for illustration only; they do not exclude other defined values or other values falling within the preferred defined ranges.

The term amorphous as used herein refers to relates to solids having short range order but not having long range order as in crystalline substances.
PVPK-30as used herein refers to polyvinyl pyrrolidone K30
Tween 30/20 as used herein refers to relate to polysorbates
Kollidone VA 64 as used herein refers to relate to vinylpyrrolidone and vinyl acetate copolymer.

Kolliphor SLS as used herein refers to relates to an alkyl sulphate on the basis of a natural, saturated, straight-chain, primary fatty alcohol.
HPBCD as used herein refers to 2-Hydroxypropyl-β-cyclodextrin (HP-β-CD)
CRYSMEB as used herein refers to low methylated beta cyclodextrin product
Avicel SMCC 90 as used herein refers to a high functionality co-processed spray-dried excipient comprising of 98 percent of microcrystalline cellulose (MCC) and 2 percent of colloidal silicon dioxide.

Cross carmellose sodium as used herein refers to internally cross-linked sodium carboxymethylcellulose
Aerosil 200 as used herein refers to hydrophilic fumed silica, Methocel E-15 LV as used herein refers to low molecular weight hydroxypropyl methylcellulose (HPMC)
Physiological pH as used herein relates to the pH of a human body.

Physiological Buffer used herein refers to buffer systems present in the body that prevent sudden and rapid changes in the pH
The phrase "stability in a packed form" used herein refers to stability of drug compositions in a suitable formulation relating to their shelf life in a form as initially prepared which has high solubility and bioavailability.

The phrase "accelerated conditions" refers to condition of elevated temperature (40° C) at 75 % relative humidity. Accelerated stability testing Studies are designed to increase the rate of chemical degradation and physical change of a drug by using exaggerated storage conditions as part of the formal stability testing programme guided by ICH (International Council for Harmonisation of Technical Requirements for Pharmaceuticals for Human Use).

Vitamin E TPGS as used herein refers to TPGS is the polyethylene glycol 1000 ester of d-α-tocopheryl succinate. It is chemically stable to heat, oxygen and light is used as solubility enhancer.

The pharmaceutical composition of the present invention comprises the compound of the Formula IV in a range of 10% to 95% , preferably 40% to 95% , more preferably 50% to 95% by weight of the composition.

The pharmaceutical compounds of the present invention comprise of one or more solubilizing agent in the range of 2% to 20% by weight of the composition, more preferably in the range of 4% to 10% by weight of the composition.

Solubilizing agents are selected from polyvinylpyrrolidone (povidone), cyclodextrin, derivative of cyclodextrin, Tween 80, Tween 20, PVPK-30, Citric acid, Kollidone VA64, Kolliphor SLS, Polyethylene glycol or mixtures, thereof.

Preferably, the solubilizing agent is povidone specifically polyvinyl pyrrolidone and cyclodextrin specifically HPBCD (hydroxypropyl beta cyclodextrin). The solubilizing agent may also be selected from sulfobutylether- cyclodextrin or CRYSMEB- Low methylated beta cyclodextrin.

Preferably, the amount of solubilizing agent in the pharmaceutical composition is in a range of 2 to 20% by weight, preferably 4 to 10% by weight.

Preferably, the pharmaceutical composition in accordance with this invention can be administered in a variety of ways.

The pharmaceutical composition containing a compound of Formula IV can be administered in therapeutically effective amounts as pharmaceutical compositions by any conventional form and route known in the art including, but not limited to: intravenous, intra-arterial, intramuscular, subcutaneous, oral, rectal, aerosol, parenteral, ophthalmic, pulmonary, transdermal, vaginal, nasal, and topical administration.

The pharmaceutical composition comprising the compounds of Formula IV can be administered by way of solid dose forms including dissolvable tablets, granule, gums, lozenges, pellets, and other forms for intraoral delivery by sublingual and buccal administration. Preferably, the claimed pharmaceutical composition is to be administered by way of oral administration, preferably in a tablet formulation.

In another embodiment, the present invention relates to a process to prepare pharmaceutical composition comprising the steps of mixing compound of formula IV and solubilizing agent. Preferably, hot melt extrusion technique, wet granulation process, dry granulation process or direct compression is used for preparing the composition. More preferably, hot melt extrusion technique is used for preparing the composition.

### Preparation:

### Process I (Hot Melt Extrusion)

The hot melt extrusion technique is an environment friendly process as it is solvent free, resulting in less effluent generation. Preferably, the solubilizing agent is polyvinylpyrrolidone when the process of preparation is hot melt extrusion.

### The process includes

Dispensing: Maintaining the humidity in the dispensing area to be not more than 50% RH. Calibrating all the balances before dispensing. Sifting: Co-sifting the compound of Formula IV and solubilizing agent, preferably polyvinylpyrrolidone through ASTM mesh # 40. Collecting the mixture and keeping it aside for further processing. Dry Blending: Blending the mixture after co-sifting in a suitable capacity blender for 10 minutes at 4 to 40 RPM (revolutions per minute), preferably 10 to 20 RPM. Extrusion Process: Passing the blended mixture through gravity feeder in twin screw barrel of suitable extrusion. Setting the parameters of zones in twin screw barrel and starting extrusion process. Then, setting suitable RPM (revolutions per minute) of feeder and screw so that the torque not be more than 50%. Checking the extruded mixture for consistency and clarity. Passing the extruded mixture through Co-mill / multi mill with 0.8 mm screen and followed by through ASTM mesh # 40.Repeating the process till all extruded mixture pass through ASTM # 40 mesh. Sifting: Co-sifting the powder extruded mixture along with desired quantity of extragranular ingredients selected from Lactose Monohydrate (Granulac^{®} 200), Silicified Microcrystalline cellulose (Avicel SMCC 90), Colloidal Silicon dioxide (Aerosil 200) or combination thereof through ASTM # 40 mesh. Blending :Mixing the above mixture in suitable capacity blender for 10 min at 16 RPM. Lubrication : Sifting lubricants such as magnesium stearate through ASTM mesh #60 and adding to the blended mixture of step 6. Mixing and lubricating the above blended mixture for 5 minutes at 16 RPM. Unloading the lubricated blend in double polythene bag lined container.

Extragranular ingredients is selected from lactose monohydrate (Granulac^{®} 200), silicified microcrystalline cellulose (Avicel SMCC 90), cross carmellose sodium (Ac Di Sol SD 711), colloidal silicon dioxide (Aerosil 200), hydroxypropyl methyl cellulose (Methocel E- 15 LV Premium) or combination thereof. The amount of extragranular ingredients is the composition is in a range of 0.2 to 40% by weight, preferably 0.2 to 20% by weight, more preferably 0.2 to 11% by weight.

Intragranular ingredients are selected from lactose monohydrate (Granulac^{®} 200), silicified microcrystalline cellulose (Avicel SMCC 90), cross carmellose sodium (Ac Di Sol SD 711), Hydroxyl Beta cyclodextrin (HPBCD), polysorbate 80, colloidal silicon dioxide (Aerosil 200), hydroxypropyl methyl cellulose (Methocel E- 15 LV Premium) or combination thereof. The amount of intragranular ingredients is the composition is in a range of 0.3 to 14% by weight.

Lubricant is selected from magnesium stearate, sodium stearyl fumarate, stearic acid, colloidal silicon dioxide etc. The amount of lubricant in the composition is in a range of 0.4 to 0.5% by weight.

Polymer is selected from Copovidone, polyvinylpolypyrrolidone (PVP), hydroxypropylcellulose (HPC), polyethylene glycol (PEG), hydroxypropyl methyl cellulose (HPMC), hypromellose acetate succinate (HPMCAS), Copovidone/vitamin E/Polyethylene glycol, Copovidone/Polysorbate 80/Colloidal silicon dioxide.

### Brief Description of Drawings

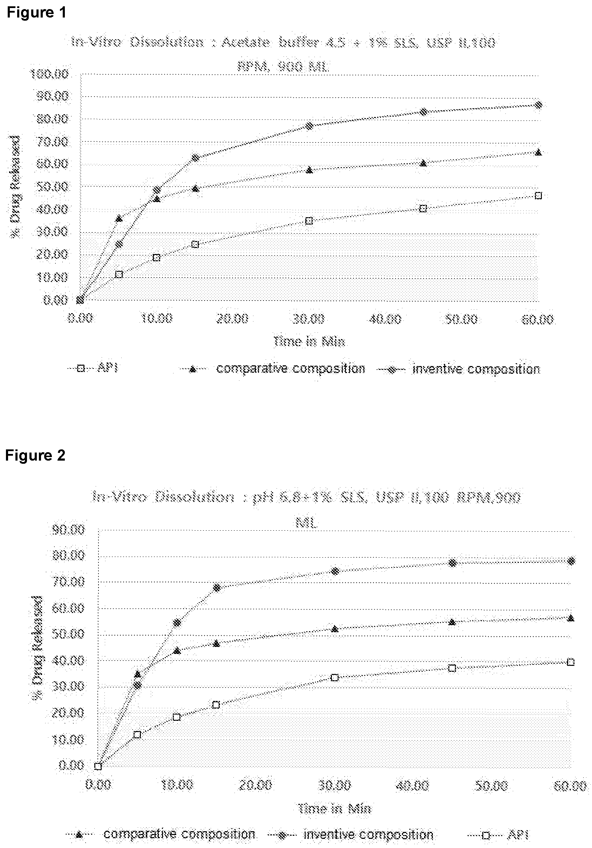

Fig. 1.: The claimed pharmaceutical composition shows superior % drug release over a period of 60 minutes. This correlates with the tabulated data of Experiment 2
Fig. 2: The claimed pharmaceutical composition shows superior % drug release over a period of 60 minutes. This correlates with the tabulated data of Experiment 3

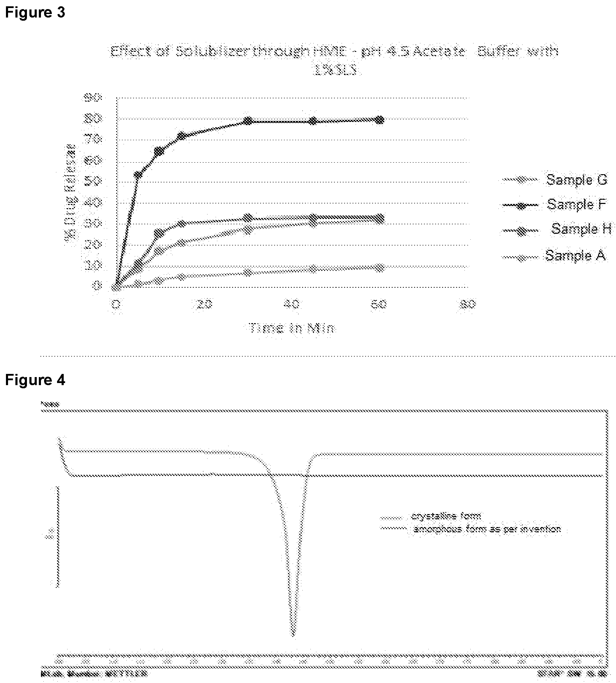

Fig 3: The claimed pharmaceutical composition shows superior % drug release over a period of 60 minutes. This correlates with the tabulated data of Experiment 4 wherein different comparative compositions comprising the compound of Formula IV have been taken.
Fig. 4: The absence of endothermic peak at 145 Deg (Melting Point of Formula V as per present invention) suggest that the Crystalline form is converted to Amorphous form in the as claimed pharmaceutical composition.

### Embodiments:

The present invention envisages an embodiment of the present invention wherein the crystalline Form of compound IV is converted to the amorphous form to make the drug more bioavailable.

In an embodiment of the present invention, the pharmaceutically acceptable salts of the compounds of Formula IV along with a solubilizing agent, form part of the claimed pharmaceutical composition
In a preferred embodiment, the compound of Formula IV is Formula V.

An embodiment of the present invention wherein the crystalline Form of compound V is converted to amorphous form to make the drug more bioavailable.

In an embodiment of the present invention the pharmaceutical composition of the present invention comprises the compound of the Formula IV in a range of 10% to 95% , preferably 40% to 95% , more preferably 50% to 95% by weight of the composition.

In another embodiment of the present invention the pharmaceutical composition of the present invention comprises the compound of the Formula IV in a range of 40% to 90% , preferably 30% to 90% , more preferably 50% to 90% by weight of the composition.

In an embodiment of the present invention of the pharmaceutical compounds of the present invention comprise of one or more solubilizing agents in the range of 2% to 20% by weight of the composition, more preferably in the range of 4% to 10% by weight of the composition.

### Process II (Wet Granulation Process)

The pharmaceutical composition of the present invention may also be arrived at by the Wet granulation Process. Preferably, the solubilizing agent is cyclodextrin, in this method.

The method comprises of the following steps:
Dispensing: Weighing compound of Formula IV along with pharmaceutically acceptable excipients. Passing intragranular ingredients through ASTM mesh #30. Preparing binder solution: Adding required quantity of water and Polysorbate 80 to the above mixture and stirring the solution and adding the cyclodextrin under continuous stirring to the above blend. The method further comprises preparing the tablet from the blend. The method comprises granulating, drying, blending, lubricating and compressing the above blend. Granulation is selected from steam granulation, moist granulation, melt granulation, hand granulation. Preferably granulation is hand granulation. Granules are dried in hot air oven for 60°C for 60min. After drying granules are passed through mesh #30 followed by adding the extragranular ingredient selected from Cross Carmellose Sodium (Ac Di Sol SD 711), Silicified Microcrystalline cellulose (Avicel SMCC 90), Colloidal Silicon dioxide (Aerosil 200) or combination thereof and pass through mesh #30 and mix for 10 min.Sifting the lubricant such as magnesium stearate through ASTM mesh 60# and adding the sifted magnesium stearate into the above mixture for 5min.Compression: In an embodiment, the lubricated blend obtained from process I and/or process II were further compressed to form tablets. The process of compression is carried out using a compression machine. Coating: In an embodiment, the tablet is coated using a coating agent. Coating of the tablets can be film coating, enteric coating or delayed release coating. Film coating agent is formed from a coating premix (Opadry II Purple 85F90093), sodium lauryl sulphate (Kolliphor SLS Fine) hydroxypropylmethylcellulose (HPMC) or polyvinyl alcohol (PVA) based or combination thereof.

The binder is selected from hydroxypropyl betadex, hydroxypropyl beta cyclodextrin (KleptoseHPB), polysorbate 80, purified water or combination thereof. The amount of binder in the composition is in a range of 2% to q.s. by weight. Tablet binder or binding agent are the substances which are added either dry or in liquid form during wet granulation to form granules or to promote cohesive compacts for directly compressed tablets.

### Formulation of tablets -Coating Process

Taking 20% of total quantity of Purified Water in the stainless-steel vessel. Dissolving weighed quantity of sodium lauryl sulphate (Kolliphor SLS Fine) under slow and continuous stirring. Adding weighed quantity of Opadry II Purple 85F90093 to the above solution of sodium lauryl sulphate, slowly under continuous stirring and stirring for 45 minutes in order to avoid formation of foam. Filtering the above solution/coating dispersion through ASTM mesh #40 and keeping dispersion under slow continuous stirring throughout process. Loading the tablets of compound of formula IV to the coating machine to achieve the weight gain of 4.00 ± 0.2 % w/w. Pre-warming the tablets to achieve the bed temperature 35°C to 45°C by rotating pan at 1-2 RPM (revolutions per minute). After getting the desired bed temperature, coating the tablet by maintaining the above-mentioned suitable parameters. After appropriate desired weight gain is achieved, drying tablets at bed temperature of not more than (NMT) 50°C, with pan at 1-2 RPM, for 15 minutes. Cooling the tablets and unloading in double polythene lined container and storing in HDPE container/IPC with silica gel as desiccant.

The present invention also envisages a composition comprising a compound of the Formula IV and a solubilizing agent as defined before for ûse in a method of treating cancer and cancer related disorders in an individual, comprising administering to the individual an effective amount of the pharmaceutical composition comprising the compounds of the Formula IV along with a solubilizing agent.

### Working Examples

**The inventors by way of experimentation have sought to arrive at the most viable pharmaceutical composition of the compounds of formula IV, which achieves significantly enhanced drug release.**

### EXPERIMENT 1

**To study the effect of different physiological pH Medium on dissolution of the compounds of Formula IV**

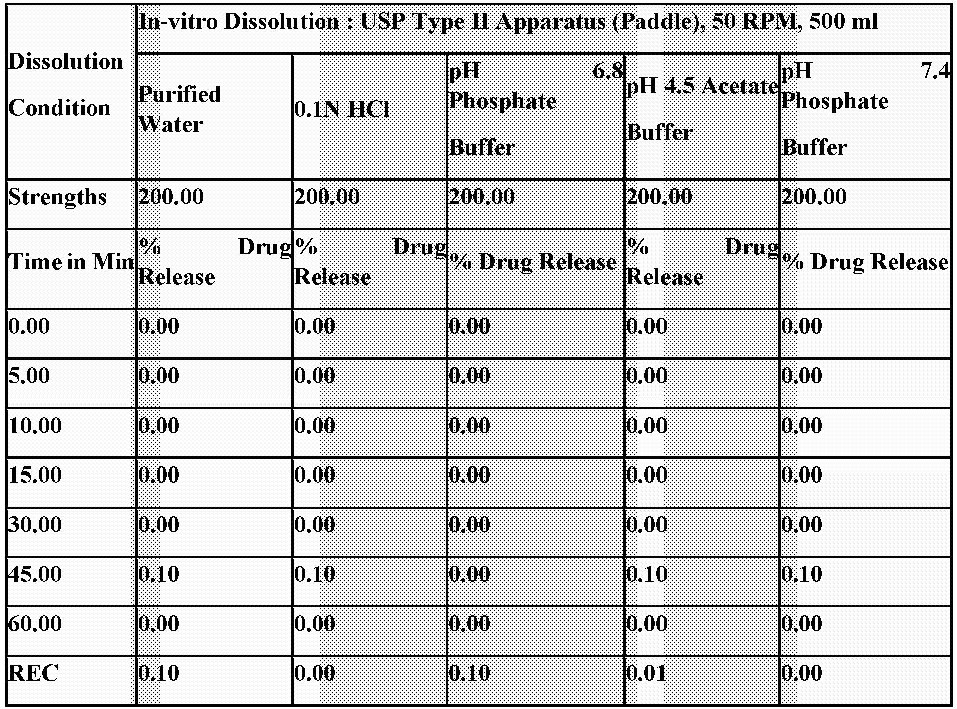

The data indicates that the compounds of Formula IV without the claimed pharmaceutical composition have no solubility in different physiological pH medium.

**A study of the dissolution profile of the claimed pharmaceutical was conducted in different physiological buffers and compared to the dissolution profile of the API and a pharmaceutical composition without the inventive composition in Experiments 2 and 3.**

### EXPERIMENT 2

The dissolution condition in this experiment was acetate buffer pH 4.5 + 1% SLS, USP II, 1000 RPM, 900 ML

| Time in minutes | API | Composition as per invention | Comparative composition without VA 64 |
|---|---|---|---|
| | | With VA 64 | |
| 0 | 0.00 | 0.00 | 0.00 |
| 5 | 11.30 | 24.90 | 36.60 |
| 10 | 19.10 | 48.70 | 45.20 |
| 15 | 25.00 | 63.00 | 49.80 |
| 30 | 35.50 | 77.40 | 57.90 |
| 45 | 41.20 | 83.90 | 61.20 |
| 60 | 47.00 | 87.30 | 66.40 |

**From the above comparison, it is observed that there is significant increase in the drug release in the claimed composition as compared to the API.**

### EXPERIMENT 3:

The dissolution condition in this experiment was acetate pH6.8 + 1% SLS, USP II, 1000 RPM, 900 ML

| Time in minutes | API | Composition as per invention | Comparative composition without VA 64 |
|---|---|---|---|
| | | With VA 64 | |
| 0 | 0.00 | 0.00 | 0.00 |
| 5 | 12.20 | 30.70 | 35.30 |
| 10 | 18.40 | 54.80 | 44.10 |
| 15 | 23.60 | 68.30 | 46.90 |
| 30 | 33.80 | 74.80 | 52.80 |
| 45 | 37.80 | 78.00 | 55.40 |
| 60 | 40.00 | 78.80 | 57.00 |

**From the above comparison, it is observed that there is significant increase in the drug release in the claimed composition as compared to the API.**

### EXPERIMENT 4

**In experiment 4, drug release for different pharmaceutical compositions of the compounds of Formula IV were studied in acetate buffer with 1%SLS (sodium lauryl sulphate). The results are produced under:**

| Composition | API | SLS | VA64(2 X) + TPGS | SLS | VA64 +TPGS + SLS | VA64 (1:2) | HPMC AS | PVA |
|---|---|---|---|---|---|---|---|---|
| Process | Hot melt extrusion | Wet granulation Water +PVPK30 | Wet granulation acetone | Wet granulation acetone | Wet granulation acetone | Hot melt extrusion | Hot melt extrusion | Hot melt extrusion |
| Sample | A | B | C | D | E | **F (as per present invention)** | G | H |
| Time in min | | | | | | | | |
| For % drug release | | | | | | | | |
| 0 | 0 | 0 | 0 | 0 | 0 | 0 | 0 | 0 |
| 5 | 1.3 | 37 | 16 | 34 | 15 | 54 | 9 | 12 |
| 10 | 3.4 | 45 | 36 | 48 | 29 | 65 | 17 | 26 |
| 15 | 4.8 | 50 | 53 | 55 | 42 | 72 | 21 | 30 |
| 30 | 6.9 | 58 | 65 | 66 | 62 | 79 | 27 | 33 |
| 45 | 8.5 | 61 | 66 | 69 | 70 | 79 | 30 | 33 |
| 60 | 9.4 | 66 | 73 | 75 | 73 | 80 | 32 | 33 |

The above results are indicative of the enhanced efficacy of the claimed composition. The pharmaceutical composition as per the present invention shows tremendous improvement in drug release from the beginning at 5, 10, 15,30, 45 and 60 minutes.

When the compound (API) is without a solubilizing agent, then the drug release is minimal. When the composition comprises the active compound along with a copolymer of vinylpyrrolidone and vinyl acetate, produced via the wet granulation, the drug release shows some improvement, but it is not sufficient to have significant effect on the final dosage. When the composition comprises SLS, it is again noted that though drug release has improved, it is similar to what is obtained when the composition is produced by wet granulation.

It can be concluded that when the composition comprises the active compound along with VA 64 in the desired wt% as per the present invention (sample F), produced by hot melt extrusion, the inventors are surprisingly achieving major improvement in the % drug release.

### Experiment 5: Accelerated, intermediate and long term stability tested for Compound V in the claimed pharmaceutical composition.

| **Sr. No.** | **Test** | **Specifications** | **Condition** | | | | | |
|---|---|---|---|---|---|---|---|---|
| | | | **Initial** | **40°C/75% RH** | | | **30°C/75% RH** | **25°C/60% RH** |
| | | | | **1 Month** | **2 Months** | **3 Months** | **3 Months** | **upto 3 Months** |
| 1 | Description | Light peach to purple colured, oval-shaped, biconvex film-coated tablets, plain on other side. | Complies | Complies | Complies | Complies | Complies | Complies |
| 2 | Assay by HPLC | Each tablet contain 90.0% of Compound of Formula V | 101.1 % | 103.3 % | 99.1 % | 98.8 % | 99.9% | 99.9 % |

| **Sr. No.** | **Test** | **Specifications** | **Condition** | | | | | |
|---|---|---|---|---|---|---|---|---|
| | | | **Initial** | **40°C/75% RH** | | | **30°C/75% RH** | **25°C/60% RH** |
| | | | | **1 Month** | **2 Months** | **3 Months** | **3 Months** | **3 Months** |
| 3 | Dissolution by HPLC Apparatus : USP Type-II, Dissolution medium : 2.0% SLS in pH 4.5 Acetate buffer, Volume: 900 mL RPM: 100, Temperature:37°C(±0.5°C) | Not less than 70%(Q) of the labeled amount of Compound V is dissolved in 60 minutes. | 93.40 % | 93.30 % | 96.10 % | 90.90 % | 97.90 % | 95.20 % |

The results show that the claimed pharmaceutical compositions have significant stability in accelerated conditions. Also, the pharmaceutical composition as claimed, comprises the compound in the amorphous form which does not degrade with time and retains its amorphous character. It is well understood by a skilled person in art that the stability of the composition of the present invention in view of the above data is extendable upto 18 months, in packed form.

### Experiment 6: Stability study:

Stability of the pharmaceutical composition in acetate buffer of 4.5+2% SLS, USP II,100RPM,900 ML for 1 month, 2 months and 3 months at different time i.e., at 0 hrs, 5 hrs, 10 hrs, 15 hrs, 30 hrs, 45 hrs and 60 hrs, respectively was studied and is represented below:

**Table 9**

| Dissolution Condition | Acetate buffer 4.5+2% SLS, USP II,100RPM,90 0 ML | Acetate buffer 4.5+2% SLS, USP II,100RPM,90 0 ML | Acetate buffer 4.5+2% SLS, USP II,100RPM,900 ML |
|---|---|---|---|
| Time in Min / Condition | 1M 40°C/75%RH | 2M 40°C/75%RH | 3M 40°C/75%RH |
| 0.00 | 0.00 | 0.00 | 0.00 |
| 5.00 | 69.10 | 72.50 | 68.10 |
| 10.00 | 77.40 | 83.70 | 75.90 |
| 15.00 | 82.60 | 88.00 | 80.80 |
| 30.00 | 88.60 | 92.40 | 88.40 |
| 45.00 | 91.90 | 94.70 | 90.30 |
| 60.00 | 93.30 | 96.10 | 90.90 |

The dissolution data on stability infers that the claimed composition is stable at accelerated condition with no significant change in drug release.

### Application

The pharmaceutical composition can be used for treatment of cancer of the breast, oral, prostate, brain, blood, bone marrow, liver, pancreas, skin, kidney, colon, ovary, lung, testicle, penis, thyroid, parathyroid, pituitary, thymus, retina, uvea, conjunctiva, spleen, head, neck, trachea, gall bladder, rectum, salivary gland, adrenal gland, throat, esophagus, lymph nodes, sweat glands, sebaceous glands, muscle, heart, or stomach; preferably for breast cancer and cancer related diseases.

## Claims

1. A pharmaceutical composition comprising the compound of Formula IV or its pharmaceutically acceptable salt thereof, 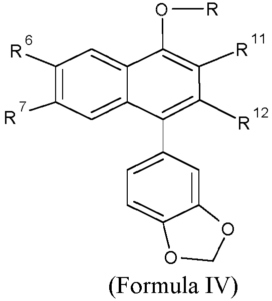
wherein R⁶ and R⁷ each independently is selected from -H, alkoxy, alkyl, , -NH₂, -NO₂, - NHCOCH₃, -CN, halogen, -OCF₃;
R¹¹ and R¹² each independently is selected from -H, or R¹¹ and R¹² can be 5- or 6-membered ring such as lactone, -C(O)O-alkyl such as -C(O)OC₂H₅; R is selected from 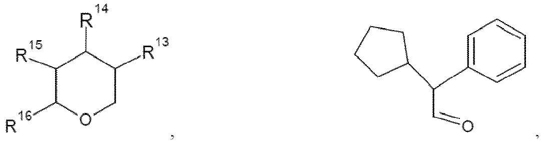 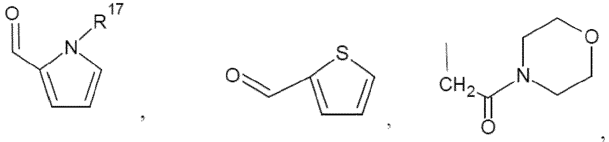 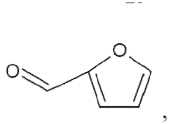 -H, -C(O)CH₂Cl, -SOO-CH₃, -SOOPh, -CH₂C(O)N(CH₃)₂, -C(O)NHPh, - C(O)NHPhOH, -C(S)NHPh, -CH₂Ph, -COAr, wherein, R¹³ is selected from -OH, - NH₂, -NHCOCH₃, X = F, Cl, Br, alkyl, acetyl, C₃-C₈ acyl group; R¹⁴ is selected from -OMe, -OH, NH₂, -NHCOCH₃, X = F, Cl, Br, alkyl, acetyl, C₃-C₈ acyl group; R¹⁵ is selected from -OMe, -OH, -H, Br, NH₂, X = F, Cl, Br, alkyl, acetyl, C₃-C₈ acyl group;
R¹⁶ is selected from -H, -CH₂OH, -OH, alkyl, alkoxy, R¹⁷ is selected from alkyl;
and a solubilizing agent is selected from polyvinylpyrrolidone, cyclodextrin, derivative of cyclodextrin, Tween 80, Tween 20, PVPK-30, citric acid, Kolidone VA64, Polyethylene glycol or mixtures thereof, further wherein the compound or a pharmaceutically acceptable salt of Formula IV, in the said pharmaceutical composition is amorphous.

2. The composition as claimed in claim 1, wherein the composition is stable at accelerated conditions of elevated temperature (40° C) at 75 % relative humidity.

3. The composition as claimed in claim 1, wherein the amount of compound of Formula IV, is in a range of 10% to 95% preferably 40% to 95%, more preferably 50% to 95% by weight of the composition.

4. The composition as claimed in claim 1, wherein the amount of solubilizing agent is in the range of 2% to 20% by weight of the composition, more preferably in the range of 4% to 10% by weight of the composition and the solubilizing agent is preferably cyclodextrin or a polyvinylpyrrolidone.

5. The composition as claimed in claim 1, comprising components selected from film coating, extragranular ingredients, intragranular ingredients, binder, lubricant, stabilizer, glidant, surface active agent, diluents, disintegrants, pH adjusting agent, polymer or mixtures thereof;
wherein the lubricant in the composition is in the range of 0.4% to 0.5% by weight of the composition and the lubricant is selected from magnesium stearate, sodium stearyl fumarate, stearic acid and colloidal silicon dioxide; or
the binder is in the range of 2% to q.s., weight of the composition and the binder is selected from hydroxypropyl betadex, hydroxypropyl betacyclodextrin, polysorbate 80, purified water or a combination thereof; or
the extra granular ingredient is in the range of 0.2% to 40%, preferably 0.2% to 20%, more preferably 0.2% to **11%** by weight of the composition and the extra granular ingredient is selected from lactose monohydrate, silicified microcrystalline cellulose, cross carmellose sodium, colloidal silicon dioxide, hydroxypropyl methyl cellulose or a combination thereof; or
the intra granular ingredient is in the range of 0.3% to 14% by weight of the composition and the intra granular ingredient is selected from lactose monohydrate, silicified microcrystalline cellulose, cross carmellose sodium, hydroxyl beta cyclodextrin, polysorbate 80, colloidal silicon dioxide, hydroxypropyl methyl cellulose or a combination thereof; or
the film coating is a mixture of coating premix, sodium lauryl sulphate and water.

6. The pharmaceutical composition as claimed in claim 1, comprising Formula V 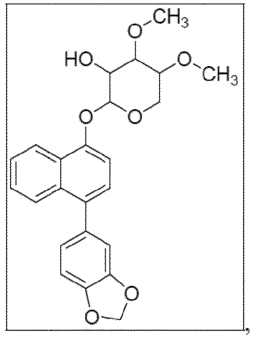 along with a solubilizing agent.

7. The pharmaceutical composition as claimed in claim 1, comprising Formula VI 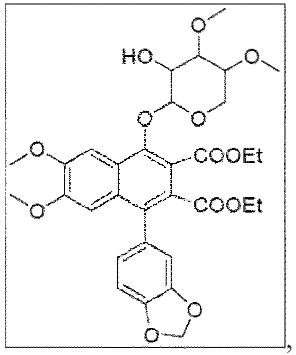 along with a solubilizing agent.

8. The pharmaceutical composition as claimed in claim 1, comprising Formula VII 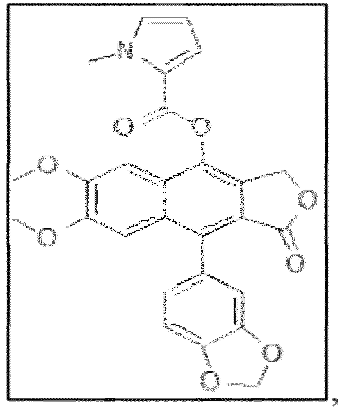 along with a solubilizing agent.

9. The pharmaceutical composition as claimed in claim 1, comprising Formula VIII 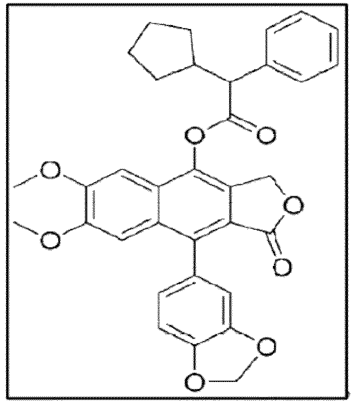 along with a solubilizing agent.

10. The pharmaceutical composition as claimed in claim 1, comprising Formula IX 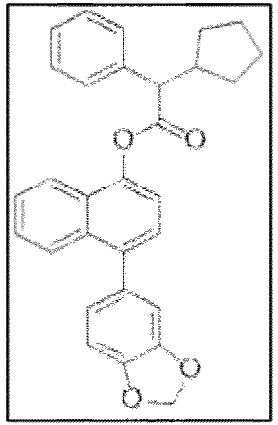 along with a solubilizing agent.

11. The pharmaceutical composition as claimed in claim 1, comprising Formula X 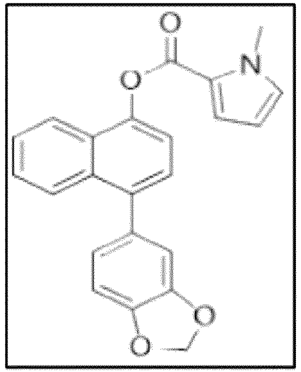 along with a solubilizing agent.

12. The pharmaceutical composition as claimed in claim 1, comprising Formula XI 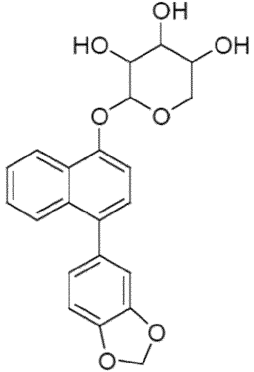 along with a solubilizing agent.

13. The composition as claimed in claim 1, wherein it is in a dosage form suitable for oral administration, preferably a tablet.

14. A method of preparing the pharmaceutical composition as claimed in claim 1,
by hot melt extrusion wherein the solubilizing agent is polyvinylpyrrolidone.

15. The pharmaceutical composition comprising any of the compound of Formula IV, for use as a medicament in the treatment of cancer or cancer related diseases.

## Patentansprüche

1. Pharmazeutische Zusammensetzung, umfassend die Verbindung der Formel IV oder ein pharmazeutisch annehmbares Salz davon, 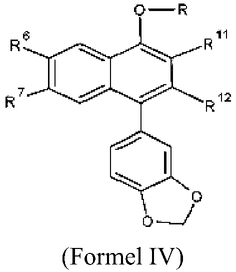
wobei R⁶ und R⁷ jeweils unabhängig ausgewählt sind aus -H, Alkoxy, Alkyl, -NH₂, - NO₂, -NHCOCH₃, -CN, Halogen, -OCF₃;
R¹¹ und R¹² jeweils unabhängig ausgewählt sind aus -H, oder R¹¹ und R¹² 5- oder 6-gliedriger Ring sein können, wie Lacton, -C(O)O-Alkyl, wie -C(O)OC₂H₅;
R ausgewählt ist aus 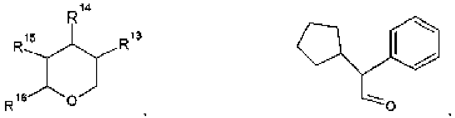 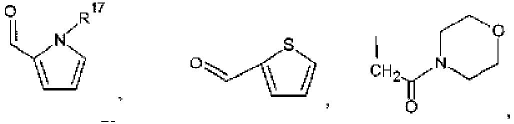 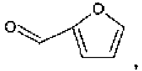 -H, -C(O)CH₂Cl, -SOO-CH₃, -SOOPh, -CH₂C(O)N(CH₃)₂, -C(O)NHPh, - C(O)NHPhOH, -C(S)NHPh, -CH₂Ph, -COAr, wobei R¹³ ausgewählt ist aus -OH, - NH₂, -NHCOCH₃, X = F, Cl, Br, Alkyl, Acetyl, C₃-C₈-Acylgruppe; R¹⁴ ausgewählt ist aus -OMe, -OH, NH₂, -NHCOCH₃, X = F, Cl, Br, Alkyl, Acetyl, C₃-C₈-Acylgruppe;
R¹⁵ ausgewählt ist aus -OMe, -OH, -H, Br, NH₂, X = F, Cl, Br, Alkyl, Acetyl, C₃-C₈-Acylgruppe; R¹⁶ ausgewählt ist aus -H, -CH₂OH, -OH, Alkyl, Alkoxy, R¹⁷ ausgewählt ist aus Alkyl;
und ein Solubilisierungsmittel, das ausgewählt ist aus Polyvinylpyrrolidon, Cyclodextrin, Derivat von Cyclodextrin, Tween 80, Tween 20, PVPK-30, Citronensäure, Kollidon VA64, Polyethylenglykol oder Mischungen davon, wobei des Weiteren die Verbindung oder ein pharmazeutisch annehmbares Salz der Formel IV in der pharmazeutischen Zusammensetzung amorph ist.

2. Zusammensetzung nach Anspruch 1, wobei die Zusammensetzung unter beschleunigen Bedingungen mit erhöhter Temperatur (40 °C) bei 75 % relativer Feuchtigkeit stabil ist.

3. Zusammensetzung nach Anspruch 1, wobei die Menge der Verbindung der Formel IV in einem Bereich von 10 Gew.% bis 95 Gew.%, vorzugsweise 40 Gew.% bis 95 Gew.%, bevorzugter 50 Gew.% bis 95 Gew.% der Zusammensetzung liegt.

4. Zusammensetzung nach Anspruch 1, wobei die Menge des Solubilisierungsmittels im Bereich von 2 Gew.% bis 20 Gew.% der Zusammensetzung, bevorzugter im Bereich von 4 Gew.% bis 10 Gew.% der Zusammensetzung liegt, und das Solubilisierungsmittel vorzugsweise Cyclodextrin oder ein Polyvinylpyrrolidon ist.

5. Zusammensetzung nach Anspruch 1, umfassend Komponenten ausgewählt aus Filmbeschichtung, extragranulären Bestandteilen, intragranulären Bestandteilen, Bindemittel, Schmiermittel, Stabilisator, Gleitmittel, oberflächenaktivem Mittel, Verdünnungsmitteln, Sprengmitteln, pH-Einstellmittel, Polymer oder Mischungen davon;
wobei das Schmiermittel in der Zusammensetzung im Bereich von 0,4 Gew.% bis 0,5 Gew.% der Zusammensetzung liegt, und das Schmiermittel ausgewählt ist aus Magnesiumstearat, Natriumstearylfumarat, Stearinsäure und kolloidalem Siliciumdioxid; oder
das Bindemittel im Bereich von 2 Gew.% bis q.s. der Zusammensetzung liegt, und das Bindemittel ausgewählt ist aus Hydroxypropylbetadex, Hydroxypropylbetacyclodextrin, Polysorbat 80, gereinigtem Wasser oder einer Kombination davon; oder
der extragranuläre Bestandteil im Bereich von 0,2 Gew.% bis 40 Gew.%, vorzugsweise 0,2 Gew.% bis 20 Gew.%, bevorzugter 0,2 Gew.% bis 11 Gew.% der Zusammensetzung liegt, und der extragranuläre Bestandteil ausgewählt ist aus Lactose-monohydrat, silicifizierter mikrokristalliner Cellulose, Crosscarmellose-Natrium, kolloidalem Siliciumdioxid, Hydroxypropylmethylcellulose oder einer Kombination davon; oder
der intragranuläre Bestandteil im Bereich von 0,3 Gew.% bis 14 Gew.% der Zusammensetzung liegt, und der intragranuläre Bestandteil ausgewählt ist aus Lactose-monohydrat, silicifizierter mikrokristalliner Cellulose, Crosscarmellose-Natrium, Hydroxyl-beta-cyclodextrin, Polysorbat 80, kolloidalem Siliciumdioxid, Hydroxypropylmethylcellulose oder einer Kombination davon; oder
die Filmbeschichtung eine Mischung aus Beschichtungsvormischung, Natriumlaurylsulfat und Wasser ist.

6. Pharmazeutische Zusammensetzung nach Anspruch 1, umfassend Formel V 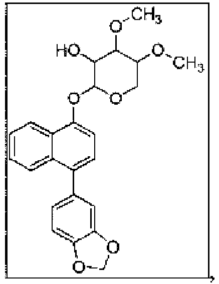 zusammen mit einem Solubilisierungsmittel.

7. Pharmazeutische Zusammensetzung nach Anspruch 1, umfassend Formel VI 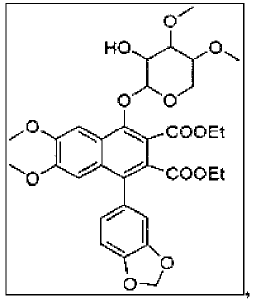 zusammen mit einem Solubilisierungsmittel.

8. Pharmazeutische Zusammensetzung nach Anspruch 1, umfassend Formel VII 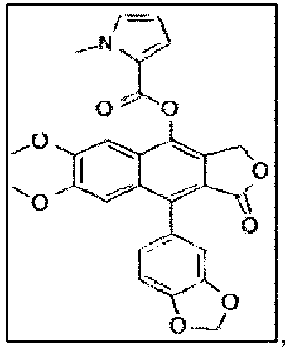 zusammen mit einem Solubilisierungsmittel.

9. Pharmazeutische Zusammensetzung nach Anspruch 1, umfassend Formel VIII 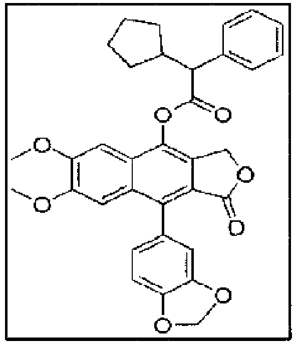 zusammen mit einem Solubilisierungsmittel.

10. Pharmazeutische Zusammensetzung nach Anspruch 1, umfassend Formel IX 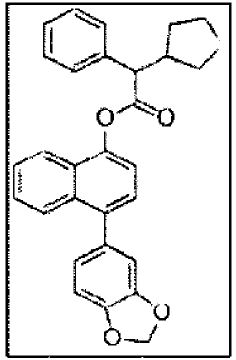 zusammen mit einem Solubilisierungsmittel.

11. Pharmazeutische Zusammensetzung nach Anspruch 1, umfassend Formel X 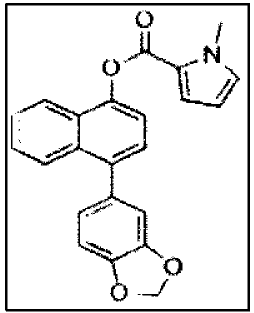 zusammen mit einem Solubilisierungsmittel.

12. Pharmazeutische Zusammensetzung nach Anspruch 1, umfassend Formel XI 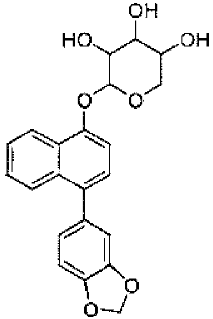 zusammen mit einem Solubilisierungsmittel.

13. Zusammensetzung nach Anspruch 1, die in einer Dosierform vorliegt, die für orale Verabreichung geeignet ist, vorzugsweise als Tablette.

14. Verfahren zum Zubereiten der pharmazeutischen Zusammensetzung nach Anspruch 1,
durch Heißschmelzextrusion, wobei das Solubilisierungsmittel Polyvinylpyrrolidon ist.

15. Pharmazeutische Zusammensetzung, umfassend jedwede von der Verbindung der Formel IV zur Verwendung als Medikament zur Behandlung von Krebs oder mit Krebs in Beziehung stehenden Erkrankungen.

## Revendications

1. Composition pharmaceutique comprenant le composé de formule IV ou un sel pharmaceutiquement acceptable de celui-ci, 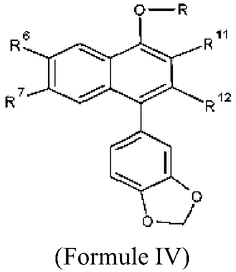
dans laquelle R⁶ et R⁷ sont chacun choisis indépendamment parmi -H, alcoxy, alkyle, -NH₂, -NO₂, -NHCOCH₃, -CN, halogène, -OCF₃ ;
R¹¹ et R¹² sont chacun choisis indépendamment parmi -H, ou R¹¹ et R¹² peuvent former un cycle à 5 ou 6 chaînons tel qu'une lactone, -C(O)O-alkyle tel que -C(O)OC₂H₅ ; R est choisi parmi 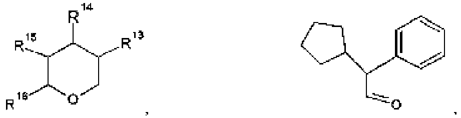 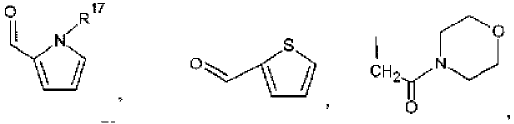 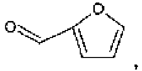 -H, -C(O)CH₂Cl, -SOO-CH₃, -SOOPh, -CH₂C(O)N(CH₃)₂, -C(O)NHPh, - C(O)NHPhOH, -C(S)NHPh, -CH₂Ph, -COAr, R¹³ étant choisi parmi -OH, - NH₂, - NHCOCH₃, X = F, Cl, Br, alkyle, acétyle, groupe acyle en C₃-C₈ ; R¹⁴ est choisi parmi -OMe, -OH, NH₂, -NHCOCH₃, X = F, Cl, Br, alkyle, acétyle, groupe acyle en C₃-C₈ ;
R¹⁵ est choisi parmi -OMe, -OH, -H, Br, NH₂, X = F, Cl, Br, alkyle, acétyle, groupe acyle en C₃-C₈ ; R¹⁶ est choisi parmi -H, -CH₂OH, -OH, alkyle, alcoxy, R¹⁷ est choisi parmi alkyle ;
et un agent solubilisant est choisi parmi la polyvinylpyrrolidone, la cyclodextrine, un dérivé de cyclodextrine, le Tween 80, le Tween 20, la PVP K-30, l'acide citrique, le Kollidon VA64, le polyéthylène glycol ou des mélanges de ceux-ci, en outre dans laquelle le composé ou un sel pharmaceutiquement acceptable de formule IV, dans ladite composition pharmaceutique, est amorphe.

2. Composition selon la revendication 1, dans laquelle la composition est stable dans des conditions accélérées de température élevée (40 °C) à 75 % d'humidité relative.

3. Composition selon la revendication 1, dans laquelle la quantité de composé de formule IV est dans une plage de 10 % à 95 %, de préférence de 40 % à 95 %, plus préférablement de 50 % à 95 % en poids de la composition.

4. Composition selon la revendication 1, dans laquelle la quantité d'agent solubilisant est dans la plage de 2 % à 20 % en poids de la composition, plus préférablement dans la plage de 4 % à 10 % en poids de la composition, et l'agent solubilisant est de préférence une cyclodextrine ou une polyvinylpyrrolidone.

5. Composition selon la revendication 1, comprenant des composants choisis parmi un enrobage pelliculaire, des ingrédients extragranulaires, des ingrédients intragranulaires, un liant, un lubrifiant, un stabilisant, un agent d'écoulement, un agent tensioactif, des diluants, des désintégrants, un agent d'ajustement du pH, un polymère ou des mélanges de ceux-ci ;
dans laquelle le lubrifiant dans la composition est dans la plage de 0,4 % à 0,5 % en poids de la composition et le lubrifiant est choisi parmi le stéarate de magnésium, le fumarate stéarylique de sodium, l'acide stéarique et le dioxyde de silicium colloïdal ; ou
le liant est dans la plage de 2 % à q.s., en poids de la composition, et le liant est choisi parmi l'hydroxypropyl bétadex, l'hydroxypropyl bêta-cyclodextrine, le polysorbate 80, l'eau purifiée ou une combinaison de ceux-ci ; ou
l'ingrédient extragranulaire est dans la plage de 0,2 % à 40 %, de préférence de 0,2 % à 20 %, plus préférablement de 0,2 % à 11 % en poids de la composition, et l'ingrédient extragranulaire est choisi parmi le lactose monohydraté, la cellulose microcristalline silicifiée, la croscarmellose sodique, le dioxyde de silicium colloïdal, l'hydroxypropylméthylcellulose ou une combinaison de ceux-ci ; ou
l'ingrédient intragranulaire est dans la plage de 0,3 % à 14 % en poids de la composition, et l'ingrédient intragranulaire est choisi parmi le lactose monohydraté, la cellulose microcristalline silicifiée, la croscarmellose sodique, l'hydroxyl bêta-cyclodextrine, le polysorbate 80, le dioxyde de silicium colloïdal, l'hydroxypropylméthylcellulose ou une combinaison de ceux-ci ; ou
l'enrobage pelliculaire est un mélange de prémélange d'enrobage, de laurylsulfate de sodium et d'eau.

6. Composition pharmaceutique selon la revendication 1, comprenant la formule V 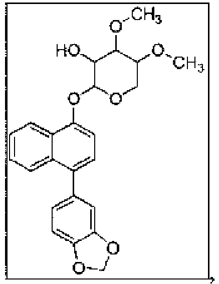 avec un agent solubilisant.

7. Composition pharmaceutique selon la revendication 1, comprenant la formule VI 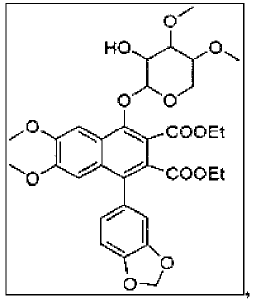 avec un agent solubilisant.

8. Composition pharmaceutique selon la revendication 1, comprenant la formule VII 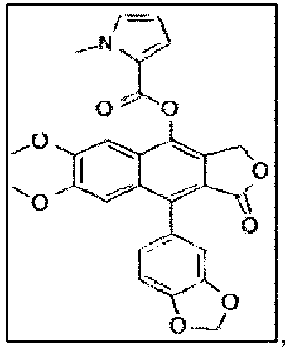 avec un agent solubilisant.

9. Composition pharmaceutique selon la revendication 1, comprenant la formule VIII 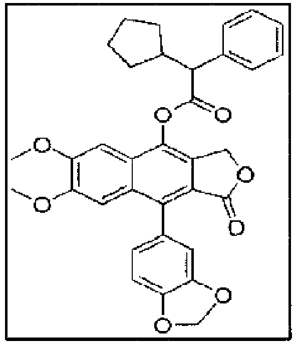 avec un agent solubilisant.

10. Composition pharmaceutique selon la revendication 1, comprenant la formule IX 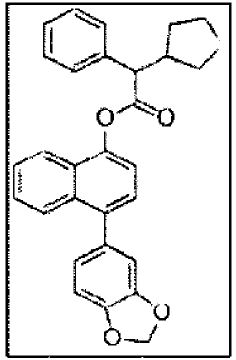 avec un agent solubilisant.

11. Composition pharmaceutique selon la revendication 1, comprenant la formule X 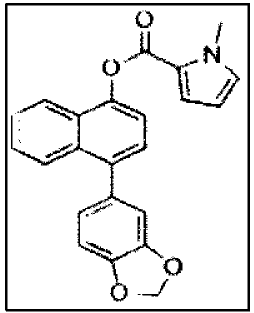 avec un agent solubilisant.

12. Composition pharmaceutique selon la revendication 1, comprenant la formule XI 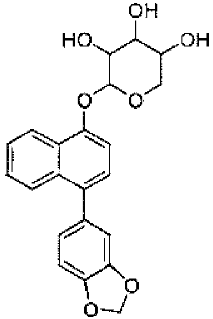 avec un agent solubilisant.

13. Composition selon la revendication 1, dans laquelle elle est sous une forme posologique adaptée à une administration orale, de préférence un comprimé.

14. Procédé de préparation de la composition pharmaceutique selon la revendication 1,
par extrusion à chaud à l'état fondu, dans lequel l'agent solubilisant est une polyvinylpyrrolidone.

15. Composition pharmaceutique comprenant l'un quelconque des composés de formule IV, pour utilisation comme médicament dans le traitement du cancer ou de maladies liées au cancer.
